# EUROPEAN PATENT SPECIFICATION

(11) **EP 2 130 906 B1**
(45) Date of publication and mention of the grant of the patent: **12.03.2014**
(21) Application number: 08157594.6
(22) Date of filing: 04.06.2008
(51) Int. Cl.: C12N 1/20

(54) **Method for the supply of growth components to cell cultures**
Verfahren zur Lieferung von Wachstumskomponenten an Zellkulturen
Procédé pour fournir des composants de croissance vers des cultures cellulaires

(43) Date of publication of application: 09.12.2009
(73) Proprietor: BioSilta Oy, Oulu (FI)
(72) Inventor: Neubauer, Peter, 13467 Berlin (DE); Vasala, Antti, 90500 Oulu (FI); Golson, Russell, Keighley, BD20 8BN Yorkshire (GB)
(74) Representative: Ganahl, Bernhard

(56) References cited:
- WO-A-2006/119867
- WO-A-2008/065254
- US-A- 3 926 723
- JEUDE M ET AL: "Fed-batch mode in shake flasks by slow-release technique" BIOTECHNOLOGY AND BIOENGINEERING, vol. 95, no. 3, October 2006 (2006-10), pages 433-445, XP002497978 ISSN: 0006-3592
- PANULA-PERALA JOHANNA ET AL: "High-cell density cultivation technique for microplates" JOURNAL OF BIOTECHNOLOGY, vol. 131, no. 2, Suppl. S, September 2007 (2007-09), page S182, XP002497984 & 13TH EUROPEAN CONGRESS ON BIOTECHNOLOGY (ECB 13); BARCELONA, SPAIN; SEPTEMBER 16 -19, 2007 ISSN: 0168-1656
- PANULA-PERALA JOHANNA ET AL: "Controlled high-cell density cultivation in shake flasks" JOURNAL OF BIOTECHNOLOGY, vol. 131, no. 2, Suppl. S, September 2007 (2007-09), page S182, XP002497985 & 13TH EUROPEAN CONGRESS ON BIOTECHNOLOGY (ECB 13); BARCELONA, SPAIN; SEPTEMBER 16 -19, 2007 ISSN: 0168-1656

## Description

### Field of the invention

The present invention relates to the field of cell and high-cell-density cultivation. More particularly the present invention relates to a method for supplying growth components to liquid cell culture media wherein the growth components are added in solid form, e.g. as tablets, and, thus, enable a controlled release when contacted with the liquid medium.

### Background of the invention

Cell cultures for the growth of microbial, fungal, higher eucaryotic and plant cells or tissues are based on cultivation of the target organisms in a liquid medium which contains all components. These media are currently prepared from powders which are dissolved in water and than sterilized, e.g. by wet heat (autoclavation) or filtration. Commonly these media contain all components which are necessary for the growth in the initial formulation in a solubilised state or in suspension. Aside from providing the nutrients, this medium also contains components which provide a buffering capacity to the medium, so that the pH is maintained in an acceptable range.

For obtaining high cell densities or continuous viability over longer periods, methods have been developed which supply parts of the medium or the whole formulation in solubilised form or as suspension discontinuously or continuously to the culture. These technologies are known as continuous culture or fed-batch cultivation.
Despite the advantage of these advanced cultivation techniques with feed addition, most cultures are performed as so-called batch cultures that are mostly continuously shaken or stirred to keep a degree of homogenicity.

Concerning the preparation and the use of these culture media the following limitations have to be taken into account:
(1) The preparation of the media is time and labour consuming. The consecutive solublisation of different components or the use of complex formulations takes time.
(2) The media are normally heat treated, which can lead to changes in volume and formulation by chemical reactions which are occuring at higher temperature
(3) High initial nutrient concentrations in media lead to long adaptation phases of the target organism, which is especially a drawback in enrichment cultures e.g. in the food or diagnostics field
(4) The supply of all growth components at one time could lead to uncontrolled consumption and results in unwanted, sometimes even toxic side metabolites, such as organic acids (e.g. acetate, lactate), alcohols (ethanol) or to limitation of oxygen in cultures which should ideally be aerobic.

Control strategies (continuous monitoring and controlling) as used for large scale cultures are not easily applicable in small shaken cultures. The setup of continuous monitoring and feeding is difficult to realize in the small scale. Non-controlled growth and insufficient aeration will bring inevitably undesirable oxygen-depletion. During oxygen limitation conditions fermentation products (acetate, CO₂, formic acid, lactic acid, ethanol, succinic acid) are formed in quantities which inhibit the growth of bacteria and impair recombinant protein processes. Some of these metabolites can be synthesized also under aerobic conditions if glucose uptake and glycolysis are faster than the capacity of the citric acid cycle. During such over-flow metabolism acetyl-CoA is transformed into acetate which is secreted to the culture medium.

Since measuring and feeding devices are not normally applicable for simple shaken cultures, alternative strategies have been developed which are based on automatic substrate delivery from polymer matrices. With such a delivery system Lübbe et al. (Appl Microbiol Biotechnol (1985) 22: 424-427) have supplied NH₄Cl in *Streptomyces clavuligerus* cultivation. Jeude et al. (Biotechnol Bioeng (2006) Vol. 95, No. 3:433-443) have used silicone elastomer (polydimethylsiloxane) disks containing glucose to create fed-batch like conditions for cultivations (see also Büchs et al. WO 2006/119867 "Fermentation method and apparatus for its implementation"). These disks can be added to cultivation vessels, but they are not integrated parts of them.

An interesting application which was limited to human cell cultures has been presented by Green and James (US3926723 "Method of controllably releasing glucose to a cell culture medium" 1975). They used small amount of soluble starch as the carbon source for cells. The horse, pig or bovine serum used in the cultivation medium provided enough catalytic activity to release gradually small amounts of glucose. Also added enzymes could be used instead of the serum enzymes. This approach used 2 g/l of starch, which in theory would support at most 1 g/l of biomass (cells). In human cell cultures no significant increase of cell number was obtained. It was not used for controlling or limiting culture growth rate, but instead it was only used to prevent accumulation of one growth-retarding compound, lactic acid.

The so far available slow-release approaches are limited 1) in scalability, 2) with regard to the amount of the delivered substrate that can be packed to the system, and 3) with regard to methods to accurately control the substrate-release, or by the use of two phase systems which are complicated to produce and limit the applicability. None of the above-described methods provides an easy solution for a high cell density growth process of target organisms.

Thus, the object of the present invention is to provide easy methods to (i) provide nutrients to a cell culture and (ii) to perform fed-batch cultivation showing high cell densities.

### Description of the invention

This object is solved by the method as claimed in claim 1. Preferred embodiments are the subject of the depending claims.

The present invention describes a method for controlling the growth of a target organism cultivated in a liquid cell culture medium, wherein a growth-limiting substrate is released in a controlled way. In particular, the present invention provides a cell culture method wherein the target organism is grown in a liquid cell culture medium to which a polysaccharide-containing tablet or pill (as these words may be used interchangeable) has been added. The polysaccharide is preferably starch.

The present invention further describes a method for packing into a tablet of pill nutrients like nitrogen compounds, which in amounts sufficient for high-cell-density would inhibit the growth of microbes. As an example concentrations of nitrogen-containing compounds (like ammonium sulphate) exceeding 200 mM inhibit the growth of E. coli. Because extra nitrogen can not be easily fed to shaking cultures in the form of ammonia (or other nitrogen-containing) solution, slow release of nitrogen from tablets provides a method to reach high cell densities and to avoid nitrogen depletion without the risk of intoxication of the organism.

Further described is a method for preventing oxygen limitation (or depletion as the words may be used interchangeably) in a cell culture by utilizing the above-mentioned slow delivery methods.

Further described is a method for controlling pH of a cell culture wherein a pH adjusting agent is released in a controlled way from the compressed tablet into the medium.

Further described is a method for supplying an inducer, activator or inhibitoring component at the beginning of the cultivation which is only released after a certain time period, e.g. sugars such as lactose, arabinose, isopropyl-β-thiogalactosides (IPTG), indole-3-acetic acid (IAA)

By integrating above mentioned components the present invention discloses a method to perform autoinduction e.g. by release of an inducer such as lactose or IPTG during a defined time interval. In this case the autoinducing agent is preferably contained in the tablet formulation and is added to the culture at the beginning.

### Brief description of the drawings

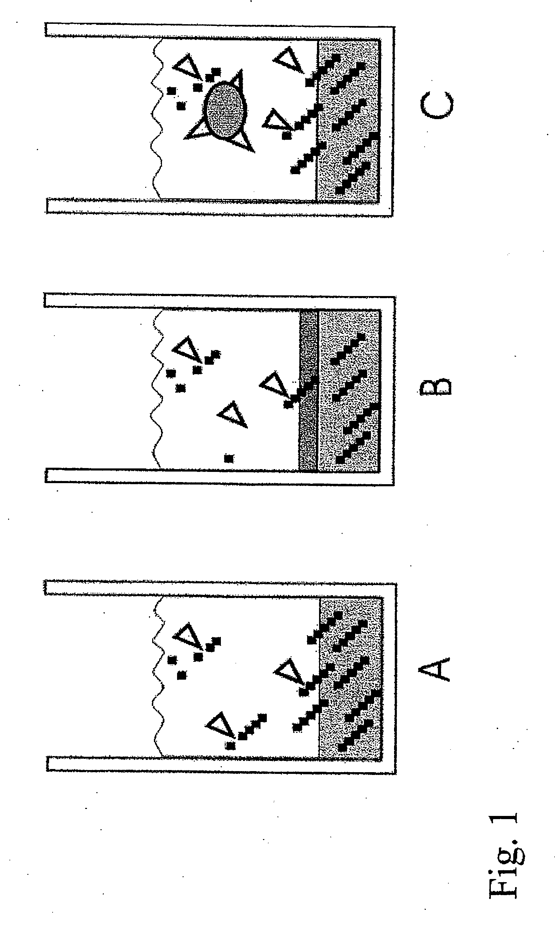

**Figure 1** shows the principle of how the method of the invention can be applied. Substrate delivery is based on a two-phase system comprising a liquid cultivation medium which can be water in the simplest case, and a compressed tablet which is added to the medium or culture at a certain phase during the process and slowly dissolves. This process can be enhanced by chemical or biological catalysators, such as enzymes which enhance the release of the components to the liquid phase.

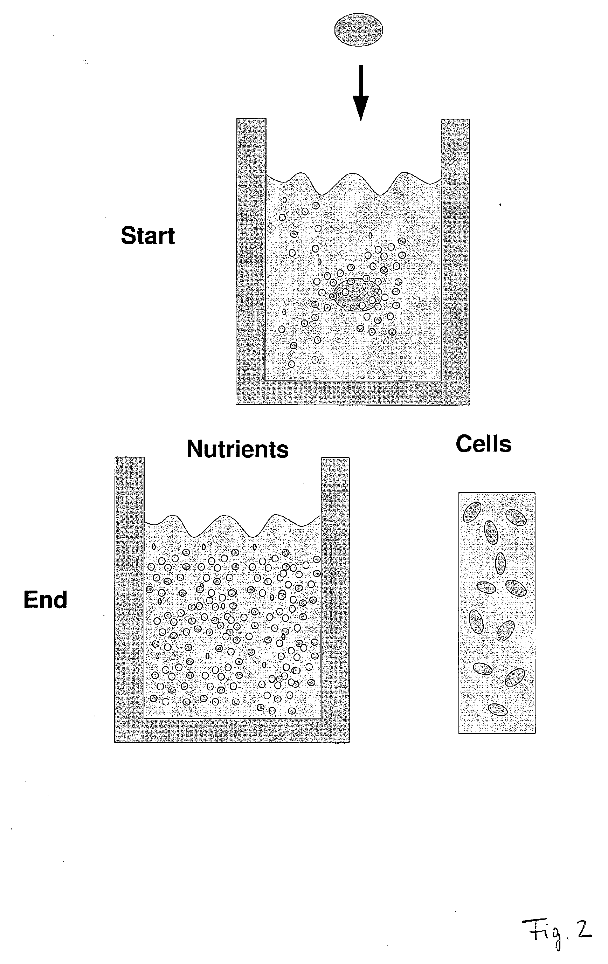

**Figure 2** shows a tablet for general medium which is fast dissoving,

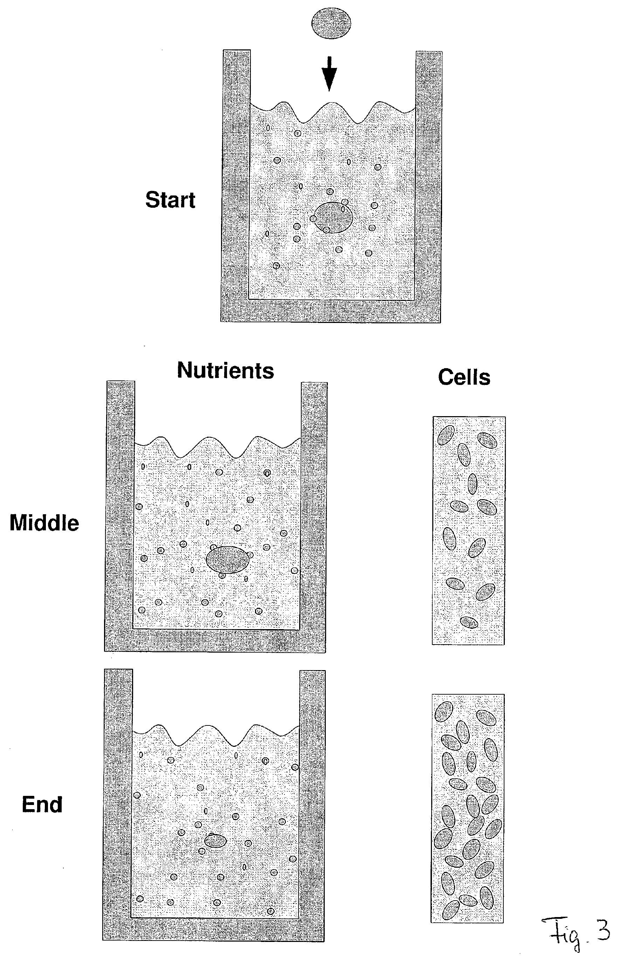

**Figure 3** shows a tablet which supplies a sugar or medium component slowly. The components can be directly used by organisms,

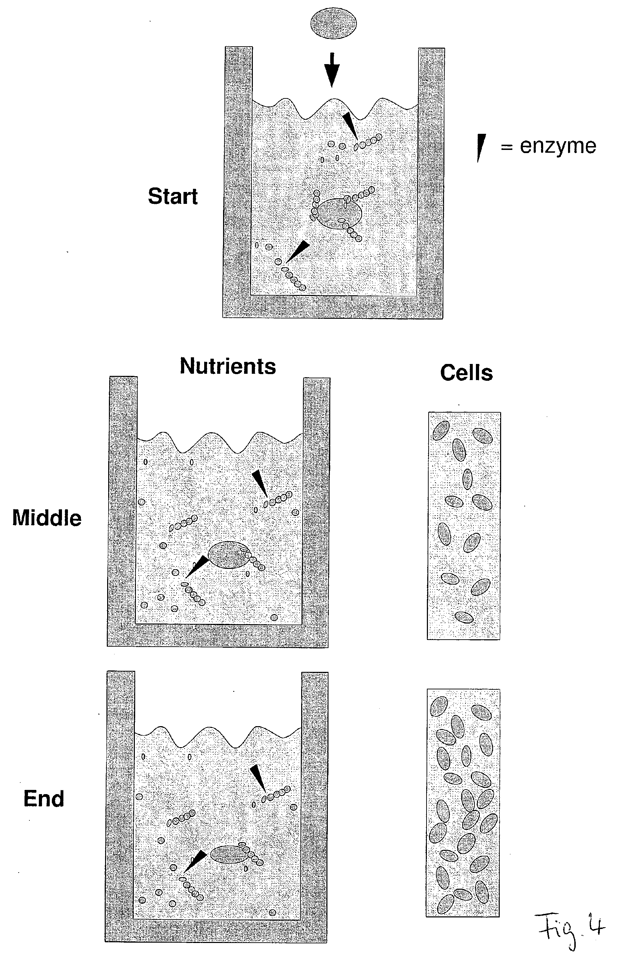

**Figure 4** shows a tablet which supplies starch which is processed into substrate by enzyme(s),

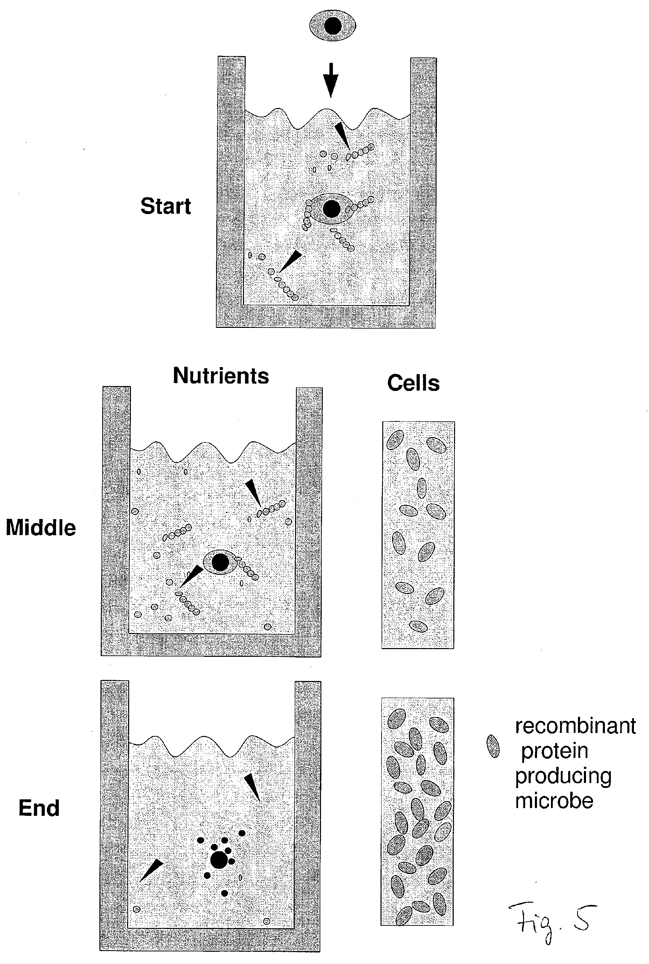

**Figure 5** shows the principle of a more-phase tablet (shell of starch and core of lactose) combining fed-batch and autoinduction. After the outer layer is dissolved, the lactose core is revealed. The change of the carbon source (diauxic shift) from glucose (enzymatically digested from starch) to lactose can thus not occur even in glucose-limited fed-batch conditions before the lactose core of the tablet is revealed.

### Detailed description of the invention

As used herein the "liquid cell culture medium" is anyone known in the art. These media and their ingredients are commercially available or applied as described in the literature and well known to a person skilled in the art. These culture media are either known as complex media which are composed of at least partly less well defined raw materials, sike extracts (e.g. yeast extract) or hydrolysates (e.g. peptone, casamino acids), or as defined media which are mixtures of chemicals with known composition. Media contain anorganic elements and mineral salts (e.g. calcium, potassium, natrium, magnesium, sulfate, bicarbonate, chloride), ammonia source(s) (ammonia, nitrate, amino acids), and carbon/energy source(s) (glucose or other sugars which may be pentamers or hexamers, starch or other sugar-based polmyers, glycerol). Additionally they may contain vitamins (e.g. ascorbic acid, riboflavin, folic acid, thiamin, vitamin A), growth factors (e.g. EGF, IGF, TGF), antibiotics (streptomycin, tetracycylin, chloramphenicol), cytokins, serum proteins (e.g. BSA, HSA), nucleosides (e.g. ribonucleosides, desoxyribonucleosides). Eventually, to provide monomeric substrates enzymes (e.g. amylases, peptidases, nucleases, proteases, peptidases, amidases) may be included in a medium. Suitable standard media for the growth of microorganismen (e.g. bacteria like E. coli, Staphylococcus, Streptococcus, Micrococcus) are for example Peptone-Yeast Extract Broth, Staphylococcus Broth, PPLO Media, Mannitol Salt Broth, Luria-Bertani Broth, DMEM, RPMI, BME, Fischer's medium or Trypticase Soy Broth. Suitable media for the growth of higher eucaryotic cells (e.g. mammalian, animal and human cells) are Isocove medium, RPMI medium, Dulbecco MEM medium, MEM medium, or F12 Medium.

As used herein "target organism" means any prokaryotic, microbial, fungal, (higher) eukaryotic and plant cell or tissue.

As used herein "high cell density" cultivation refers to a cultivation which yields a high number of target organism cells. This requires a high amount of nutrients. Which density of a target organismen can be reached and would be considered to be high density depends on the kind and type of organismen. In the present invention the growth of e.g. bacterial cells over OD₆₀₀=20 corresponding to 20 g/l of cell wet weight and of mammalian cells over 2x10⁶ cells/ml would be considered to be "high density". In general, "high density" can be defined as the cell density that can be reached by controlled supply of growth components without toxicating the target organism.

The method of the invention is scalable over a wide range of volumes and can be applied on several different cultivation equipment and volumes. For example, the cultivation vessel may a microtiter plate, glass vial, shake flask, Falcon tube, Ep-pendorf cup, laboratory fermenter or industrial production fermeter. There are no limitations with regard to size and volume.

As used herein "batch cultivation" refers to a cultivation process where all components are added at the beginning of the cultivation. No external feeding occurs during the culturing time. As used herein "fed-batch" refers to a cultivation where nutrients are gradually fed during the cultivation. As used herein "substrate-limited fed-batch" refers to a cultivation method where the growth of the target organism is controlled with one limiting nutrient, e.g. by the use of glucose as the only carbon source.

The inventors have recognized that glucose can be easily provided to the cell culture medium in form of a compressed polysaccharide-containing tablet or pill which may also contain further ingredients, like mineral salts, growth factors, proteins, etc. In a preferred embodiment the polysaccharide-containing tablet further contains at least one polysaccharide-digesting enzyme, like amylase and/or glucoamylase. If the digesting enzyme is not contained in the polysaccharide-containing tablet a suitable amount may be preferably contained in the liquid culture medium to digest the polysaccharide. It is preferred that the end-concentration of glucose in the culture medium is below 1 g/l, preferably between 0 and 200 mg/l

The tablet manufacturing for slow release of different compounds is a well-established technology in the pharmacy. Starch itself can be an ingredient forming the backbone of the tablet. Other materials that improve the structure and properties of a tablet may include components like microfibrous cellulose, PEG, clay, alginates, gums, crosslinked polymers, etc. Various other natural polymers like alginates (combined with other polymers) can be used as raw materials.

Additional functional compounds can be buried inside the tablet ("coated") with the aim of being released when the tablet is decomposed. One of these additional functional compounds is a pH-control or pH-adjusting compound that preferably dissolves in pH < 6.5. A pH-elevating chemical can be for example a compound that releases ammonia. This may be an ammonis salt (e.g. ammonia sulphate) or a compound that releases ammonia after treatment by some enzymes (e.g., amidases). This may be achieved by enzymatically digesting a nitrogen-containing polymeric material. An example of such a polymeric material is polyacrylamide from which amino groups can be enzymatically released by amidases, or gelatine which is gradually digested by proteases, peptidases or amidases. Release of ammonia from such compounds can elevate the pH and facilitate the control of the pH. In addition, it provides a source of nitrogen. Possibly also buffer systems (e.g. such as HEPES, MOPS) can be incorporated into the tablet.

The method of the present invention can be controlled by optimizing several parameters, such as enzyme concentration, enzymatic activity and nutrient concentration. Also during the cultivation process the parameters may be easily observed by measuring the cell density and changed if toxication of the target organismen is immenent (e.g. by oxygen depletion).

Another preferred ingredient of the tablet is IPTG or lactose which can be used as an inducer for recombinant bacteria carrying the lac-operon. In this regard lactose would have a double function within the tablet: it would be simultaneously a binder within the tablet and an inducer for the target microorgismen. However, also other sugars might be added, e.g. arabinose, rhamnose, or sucrose. It is preferred that the inducer forms the core of the tablet or pill and the polysaccharide (e.g. starch) form the surrounding material around the core.

Additionally the tablet or pill may contain vitamins (e.g. ascorbic acid, riboflavin, folic acid, thiamin, vitamin A), growth factors (e.g. EGF, IGF, TGF), antibiotics (streptomycin, tetracycylin, chloramphenicol), cytokins, serum proteins (e.g. BSA, HSA), nucleosides (e.g. ribonucleosides, desoxyribonucleosides). Eventually, to provide monomeric substrates enzymes (e.g. amylases, peptidases, nucleases, proteases, peptidases, amidases) may be included.

The operational principle of the tablet product is as follows:
- Starch acts as a glucose source. Controlled and optimal glucose release can be obtained by suitable enzyme dosing (irrespective to the concentration of starch in the medium).
- By formulating starch into tablet (with or without additional tablet backbone materials), a convenient method (and easy-to-handle customer product) to deliver starch into liquid medium is achieved. The starch release rate can be properly tailored by using different starch qualities (polymer lenghts, different proportions of amylose and amylopectin; starches from different origins, such as from potatoe, corn, rice etc.) and their extracts and additional tablet- or pill-forming materials (celluloses, alginates etc.).
- Starch release from the tablet to the liquid cell culture medium is affected by passive diffusion, but preferably by the enzymatic action of amylases
- Dissolving of starch from the tablet also liberates other ingredients of the tablet to the medium

In the present invention knowledge from the pharmaceutical area in the field of tablet formulations is adapted to the field of biocultivation. Tablet formulations have been so far not described for the application in the field of cell cultivation. The invention is to compress starch and optionally other components and nutrients in the form of pills or tablets and supply them to sterile water, to provide the general nutrients and/or specific components of the growth medium. Tablet formulation have the following specific advantages:
1. The medium can be sterilized by radiation and is not subjected to heat
2. Non-soluble media components which otherwise would be heat treated, as they cannot be filtered, can be integrated in tablets.
3.The release rate of the substrate can be controlled by the formulation (either fast release for providing the initial nutrients, or slow releae to performing a mode of fed-batch priniciple).
4. By encapsulation the release rate can be controlled in a way that the release only starts after a certain period, which allows the design of specific process phases, e.g. supply of inducers or activators at a certain state of the cultivation without the need of interrupting the culture.
5.Tablets are very easy to use and as they are supplied in sterile form can be used by simply adding them to presterilised water. Consequently formulations will not change, as they do e.g. after autoclavation (chemical modifications, volume losses, pH change) or after filtration (exlosure of components, filter clogging)
6. Especially tablets also can contain freeze-dried bioorganic components, such as (i) enzymes to e.g control cell growth, or even (ii) whole cells to perform cultivation directly after inoculating the water with a tablet without any further inoculation.

Thus, the present invention provides a method to perform a fed-batch cultivation with otherwise non-controlled shaken microbial cultures in order to reach high cell densities (i.e. considerably higher than the cell densities obtained by batch-cultivations) without external feeding. The growth-limiting substrate glucose is obtained by enzymatic release from starch which is contained in the tablet. An advantage of the method of the present invention is that the synthesis of growth-limiting metabolites in the cell culture can be restricted. This is obtained by preventing excessive substrate feed (the cause of over-flow metabolisms) and non-controlled growth (the cause of oxygen depletion). The method of the invention is also useful for controlling the pH of the cell culture. Another advantage is that pumps or other external devices are not required and therefore the cultivation system can be simple and cost-efficient.
The invention is further described in more detail in the following examples which should not be considered to limit the scope of the invention.

### Examples

### Example 1: Controlled growth of Escherichia coli to obtain high cell-densities

E. coli W3110 is cultivated in 1 L shake flasks containing 100 mL of sterile mineral salt medium (as described by Neubauer et al., J. Biotechnol. 43, 195-204 but with double ammonia concentration) at 37°C with a rotating shaker (150 rpm, 25 mm ampitude). The basic medium does not contain any compound which can be directly used as a carbon source. 3 tablets compressed each of 0.8 g of soluble potatoe starch are added to the cultures. Glucoamylase is added to the culture at a concentration which releases low amounts of glucose. The optimal concentration is obtained by testing different dilutions. During the cultivation, starch is slowly dissolving from the tablet to the cultivation medium, where the enzyme releases glucose from starch. The enzymatic feeding of glucose from starch provides a method to control the growth rate and respiratory activity of bacteria. Synthesis of growth-retarding products of over-flow metabolism (substrate overfeed) and anaerobic metabolism are therefore minimized. The culture showed a linear growth until an optical density (OD₆₀₀) of 25 to 30 which was reached after about 24 to 48 hours depending on the concentration of the enzyme.

### Example 2. Addition of nitrogen from a tablet to the medium

The culture performed is the same as in example 1 but with the ammonia concentration as published by Neubauer et al., J. Biotechnol. 43, 195-204. For continuous control of the ammonia concentration and a better pH control, the 3 tablets, additionally to the starch (see example 1) contained each 0.04 mg of ammonia sulfate. Growth to similar cell densities as in example 1 was obtained in the same time interval.

### Example 3. High cell-density cultivation followed by autoinduction of a recombinant gene.

Expression of a recombinant gene can be turned on by adding an inducer component like lactose into the cultivation medium. Lactose will be taken inside bacteria only when glucose is exhausted. When using glucose-limited fed-batch, the pre-sense of lactose directly in the medium may cause leaky expression of the recombinant gene. Therefore, encapsulation of lactose into the core of a tablet ensures that lactose-induced expression occurs at a wanted cell-density. When the starch-coating has dissolved, the lactose-containing core of the tablet is revealed, and diffusion of starch to the medium starts. If the amount of free glucose is then very low, E. coli starts using lactose as a carbon source (phenomenom known as diauxic shift). Lactose taken into the cell inactivates lacl repressor protein, and expression of the recombinant gene can start.

As an example of this starch tablets with a lactose core containing in total 2.5 g of soluble potatoe starch and 2 g lactose were compressed and used in a cultivation of E. coli BL21 containing a pET-plasmid encoding for a recombinant protein (trio-sephosphate isomerase, TIM) The culture was prepared as described in Example 1, but the medium additionally contained 100 mg/l of ampicilline and the culture was performed at 25°C. The culture was incubated for 36 to 48 hours. After this time a high expression of recombinant TIM was observed.

This example demonstrates a facile combination of high cell-desity cultivation and an autoinduction system which has a low hands-on time. By intelligent design, it is possible to define the induction cell-density.

### Example 4. Use of starch tablets in bioreactors

Fed-batch is the desired technology for bioreactor cultivations. Typically very concentrated glucose solutions are used for feeding in order to minimize the dilution of the bioreactor content. Pumping of concentrated glucose solutions creates zones of different nutrient contents; some areas contain excess glucose which induces overflow metabolism, while in other areas cells may suffer from starvation, which occurs in large-scale bioreactors, but can be simulated in laboratory-scale reactors by using very low stirrer speeds (e.g. 100 rpm). By the use of starch releasing tablets (prepared as described above) together with glucoamylase which is supplied ot the medium, more even glucose feeding can be applied and the bioreactor effect can be minimised. Therefore the tablets are supplied at the time when the glucose feeding is started or the tablets replace the glucose feeding. In the example the initial glucose concentration is 10 g/l and the total starch concentration supplied in form of tablets to the reactor after the initially added glucose was consumed is 30 g/l.

### Example 5. Use of starch tablets in cell cultures.

Animal or plant cells cultivated in liquid media need small amounts of glucose, but produce growth-limiting metabolites like lactic acid in the presence of excess glucose. By using tablets or granules which slowly release starch in combination with starch-degrading enzyme, accurate glucose feeding system can be applied.

In the present example hybridoma cells are cultivated in spinner flasks in Eagle's MEM medium containing 750 mg/l sodium bicarbonate, 4 mM glutamine, and 5% fetal bovine serum as well as 20 mM HEPES buffer and 1mM sodium pyruvate. The pH value is adjusted to 7.15 with NaOH. Tablets which were composed of starch and glutamine (each 500-800 µmol) were added when the initial glucose concentration decreased below 1 mM (30-50 h of cultivation). The glucoamylase concentration is adapted so that 1.5-2.5 µmol/(lh) glucose are released.

## Claims

1. A method for controlling the growth of a prokaryotic, microbial or eukaryotic cell cultivated in a liquid cell culture medium, wherein an enzyme controllably releases the growth-limiting substrate glucose from a starch-containing tablet added to the liquid medium.

2. The method of claim 1, wherein the tablet further contains at least one component of the group consisting of a pH adjusting agent, an inducer, activator and/or inhibitoring component, wherein the pH adjusting agent is selected from the group consisting of buffer systems and compounds that release ammonia and the inducer, activator and/or inhibitoring component is selected from the group consisting of lactose, arabinose, IPTG and IAA.

3. The method of claim 1 or 2, wherein the liquid cell culture medium is LB broth, Peptone Yeast Extract Broth, DMEM, MEM, RPMI medium or F12 medium.

4. The method of any of the preceding claims, wherein the enzyme is a starch-digesting enzyme that is contained in the liquid cell culture medium.

5. The method of claim 2, wherein the inducer is lactose or IPTG.

6. The method of any of the preceding claims, wherein the enzyme controllably releases the growth-limiting substrate glucose from starch by controlling the enzyme concentration and/or enzyme activity.

7. The method of claim 4, wherein the starch-digesting enzyme is amylase.

8. The method of any of the preceding claims, wherein the tablet contains at least one of the components selected from vitamins, growth factors, antibiotics, cytokins, serum proteins, nucleosides and enzymes.

9. The method of claim 8, wherein the enzyme is selected from proteases, peptidases, nucleases and amidases.

10. A high-cell-density fed-batch technology cultivation kit for controlling the growth of a prokaryotic, microbial or eukaryotic cell cultivated in a liquid medium, wherein it comprises a liquid cell culture medium and a starch-containing tablet, wherein an enzyme releases with a controlled release rate the growth-limiting substrate glucose from starch from the tablet added to the liquid medium.

## Patentansprüche

1. Verfahren zur Wachstumskontrolle einer in einem flüssigen Zellkulturmedium kultivierten prokaryotischen, mikrobiellen oder eukaryotischen Zelle, wobei ein Enzym das wachstumslimitierende Substrat Glucose aus einer dem flüssigen Medium zugegebenen stärkeenthaltenden Tablette kontrolliert freisetzt.

2. Verfahren nach Anspruch 1, wobei die Tablette weiterhin mindestens einen Bestandteil aus der Gruppe bestehend aus einem pH-anpassenden Agens, einem Induktor, Aktivator und/oder inhibitorischen Bestandteil enthält, wobei das pH-anpassende Agens aus der Gruppe ausgewählt ist, die aus Puffersystemen und Ammoniak freisetzenden Verbindungen besteht, und der Induktor, Aktivator und/oder inhibitorische Bestandteil aus der Gruppe ausgewählt ist, die aus Lactose, Arabinose, IPTG und IAA besteht.

3. Verfahren nach Anspruch 1 oder 2, wobei das flüssige Zellkulturmedium LB-Medium, Pepton-Hefeextrakt-Medium, DMEM-, MEM-, RPMI-Medium oder F12-Medium ist.

4. Verfahren nach einem der vorangegangenen Ansprüche, wobei das Enzym ein stärkeverdauendes Enzym ist, das in dem flüssigen Zellkulturmedium enthalten ist.

5. Verfahren nach Anspruch 2, wobei der Induktor Lactose oder IPTG ist.

6. Verfahren nach einem der vorangegangenen Ansprüche, wobei das Enzym das wachstumslimitierende Substrat Glucose durch Kontrollieren der Enzymkonzentration und/oder Enzymaktivität aus Stärke kontrolliert freisetzt.

7. Verfahren nach Anspruch 4, wobei das stärkeverdauende Enzym Amylase ist.

8. Verfahren nach einem der vorangegangenen Ansprüche, wobei die Tablette mindestens einen der Bestandteile ausgewählt aus Vitaminen, Wachstumsfaktoren, Antibiotika, Cytokinen, Serumproteinen, Nucleosiden und Enzymen enthält.

9. Verfahren nach Anspruch 8, wobei das Enzym aus Proteasen, Peptidasen, Nucleasen und Amidasen ausgewählt ist.

10. Hohe-Zelldichte-Fed-Batch-Technologie-Kultivierungskit zur Wachstumskontrolle einer in einem flüssigen Medium kultivierten prokaryotischen, mikrobiellen oder eukaryotischen Zelle, wobei es ein flüssiges Zellkulturmedium und eine stärkeenthaltende Tablette umfasst, wobei ein Enzym das wachstumslimitierende Substrat Glucose aus Stärke aus einer dem flüssigen Medium zugegebenen Tablette mit einer kontrollierten Freisetzungsrate freisetzt.

## Revendications

1. Procédé pour contrôler la croissance d'une cellule procaryote, microbienne ou eucaryote cultivée dans un milieu de culture de cellules liquide, dans lequel une enzyme libère de manière contrôlée le glucose de substrat limitant la croissance d'un comprimé contenant de l'amidon ajouté au milieu liquide.

2. Procédé selon la revendication 1, dans lequel le comprimé contient par ailleurs au moins un composant du groupe composé d'un agent d'ajustement du pH, un inducteur, un activateur et/ou un composant inhibiteur, dans lequel l'agent d'ajustement du pH est choisi parmi le groupe composé de systèmes tampons et de composés qui libèrent de l'ammoniac et l'inducteur, l'activateur et/ou le composant inhibiteur est choisi parmi le groupe composé de lactose, d'arabinose, d'IPTG et d'IAA.

3. Procédé selon la revendication 1 ou 2, dans lequel le milieu de culture de cellules liquide est un bouillon de LB, un bouillon d'Extrait de Levure-Peptone, un milieu DMEM. MEM, RPMI ou un milieu F12.

4. Procédé selon l'une quelconque des revendications précédentes, dans lequel l'enzyme est une enzyme digérant l'amidon qui est contenue dans le milieu de culture de cellules liquide.

5. Procédé selon la revendication 2, dans lequel l'inducteur est lactose ou IPTG.

6. Procédé selon l'une quelconque des revendications précédentes, dans lequel l'enzyme libère de manière contrôlée le glucose de substrat limitant la croissance de l'amidon en contrôlant la concentration d'enzyme et/ou l'activité enzymatique.

7. Procédé selon la revendication 4, dans lequel l'enzyme digérant l'amidon est amylase.

8. Procédé selon l'une quelconque des revendications précédentes, dans lequel le comprimé contient au moins l'un des composants choisis parmi les vitamines, les facteurs de croissance, les antibiotiques, les cytokines, les protéines de sérum, les nucléosides et les enzymes.

9. Procédé selon la revendication 8, dans lequel l'enzyme est choisie parmi les protéases, les peptidases, les nucléases et les amidases.

10. Kit de culture selon la technologie à alimentation discontinue à haute densité cellulaire pour contrôler la croissance d'une cellule procaryote, microbienne ou eucaryote cultivée dans un milieu liquide, dans lequel il comprend un milieu de culture de cellules liquide et un comprimé contenant de l'amidon, dans lequel une enzyme libère à un taux de libération contrôlée le glucose de substrat limitant la croissance de l'amidon de la tablette ajoutée au milieu liquide.
